# EUROPÄISCHE PATENTANMELDUNG

(11) **EP 1 160 241 A2**
(43) Veröffentlichungstag der Anmeldung: **05.12.2001**
(21) Anmeldenummer: 01113350.1
(22) Anmeldetag: 01.06.2001
(51) Int. Cl.: C07D 215/56

(54) **Verfahren zur Herstellung von Chinolon-3-carbonsäuren**

(30) Priorität: 03.06.2000 DE 10026903
(71) Anmelder: CPC Cellular Process Chemistry GmbH, 60343 Frankfurt (DE)
(72) Erfinder: Schwalbe, Thomas Dr., 61118 Bad Vilbel (DE); Taghavi-Moghadam, Shahriyar Dr., 63263 Neu-Isenburg (DE); Rüger, Reinhold Dr., 63322 Rödermark (DE)

(57) **Zusammenfassung**

Die Erfindung betrifft ein verbessertes Verfahren zur Herstellung der Verbindungen der Formel I, 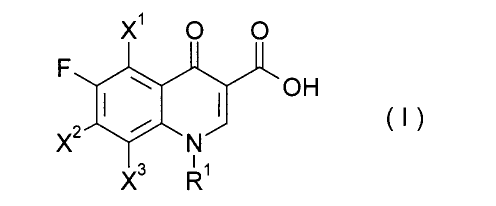 worin X¹, X², X³ und R¹ die in den Ansprüchen angegebene Bedeutung aufweisen, wobei mindestens eine der Umsetzungen kontinuierlich geführt wird und die jeweiligen Edukte gegebenenfalls in Gegenwart eines inerten Verdünnungsmittels in einer Mischzone miteinander gemischt werden und in einer Reaktionszone gegebenenfalls unter erhöhtem Druck und bei im wesentlichen konstanter Temperatur gehalten werden, sowie ein Mikroreaktorsystem zur Durchführung des erfindungsgemäßen Verfahrens, welches mindestens zwei miteinander verbundene Mikroreaktoren und ein dazwischengeschaltetes Verweilzeitmodul und/oder Reinigungsmodul umfasst.

## Beschreibung

Die Erfindung bezieht sich auf ein verbessertes Verfahren zur Herstellung von Chinolon-3-carbonsäuren der Formel I, 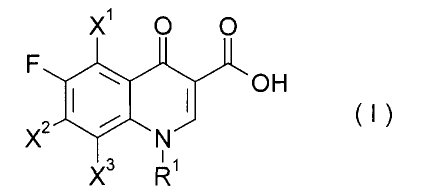 worin
X¹ für Wasserstoff, Halogen, C₁-C₆-Alkyl oder Amino steht,
X² für Halogen, C₁-C₆-Alkyl, gegebenenfalls substituiertes C₆-C₁₀-Aryl, gegebenenfalls substituiertes 5- oder 6-gliedriges Stickstoffhaltiges Heteroaryl steht, oder eine Gruppe der Formel ZR² bedeutet, wobei Z für O, S oder NR3 steht, und R² und R³ jeweils unabhängig voneinander C₁-C₆-Alkyl, gegebenenfalls substituiertes C₆-C₁₀-Aryl, gegebenenfalls substituiertes 5- oder 6-gliedriges Stickstoffhaltiges Heteroaryl bedeuten, oder R² und R³ zusammen mit dem eingeschlossenen Stickstoffatom einen gegebenenfalls substituierten heterocyclischen Rest mit 3 bis 8 Ringgliedern und gegebenenfalls einem oder zwei weiteren Hetetoatomen ausgewählt aus der Gruppe O, S und N bilden,
X³ für Wasserstoff, Halogen, C₁-C₆-Alkyl, C₁-C₆-Alkoxy oder Amino steht, und
R¹ für C₁-C₆-Alkyl, C₁-C₆-Haloalkyl, C₃-C₈-Cycloalkyl, gegebenenfalls substituiertes C₆-C₁₀-Aryl, oder gegebenenfalls substituiertes 5- oder 6-gliedriges Stickstoffhaltiges Heteroaryl steht.

Die Verbindungen der Formel I sind bekannte pharmazeutische Wirkstoffe mit antibakterieller Wirkung. Daher besteht ein Bedarf an wirtschaftlichen Herstellungsverfahren zur Produktion dieser Verbindungen.

Die europäische Patentanmeldung EP 0 167 763 schlägt vor, solche Verbindungen, worin X² einen Stickstoffhaltigen Heterocyclus bedeutet, in einem mehrstufiges Verfahren durch Cyclisierung von 2-(2,3,4,5-Tetrahalobenzoyl)-3-cyclopropylaminoacrylaten, anschließender Verseifung und anschließende Einführung des Substituenten in der 7-Position (Formel I: X²) herzustellen. Aufgrund der niedrigen Gesamtausbeute und der erforderlichen Reaktionszeiten ist dieses Verfahren für die großtechnische Herstellung der Verbindungen der Formel I wenig geeignet.

Die europäische Patentanmeldung EP 0 275 971 beschreibt ein Verfahren zur Herstellung der Verbindungen der Formel I, worin X² einen Stickstoffhaltigen Heterocyclus bedeutet, durch ein mehrstufiges Verfahren ausgehend von 2-(2,3,4,5-Tetrahalobenzoyl)-essigsäureestern. Aufgrund der bei der Cyclisierung erforderlichen starken Basen, wie zum Beispiel Natriumhydrid, und der niedrigen Gesamtausbeute ist dieses Verfahren für die großtechnische Herstellung der Verbindungen der Formel I nicht geeignet.

In der europäischen Patentanmeldung EP 0 657 448 wird ein mehrstufiges Eintopfverfahren zur Herstellung von 7-substituierten Chinolon-3-carbonsäuren ausgehend von Tetra- oder Pentahalobenzoylchloriden offenbart. Dieses umfasst die Umsetzung des Säurechlorids mit einem 2-Aminoacrylat, Cyclisierung in Gegenwart einer Base, Einführung des Restes in 7-Stellung und anschließende Verseifung. Dabei fällt das Endprodukt als Feststoff in Form eines inneren Salzes an. Als nachteilig bei diesem Verfahren erweisen sich die bei den Reaktionen gebildeten Mengen an weiteren Feststoffen in der Reaktionsmischung, die ein Übertragen des Verfahrens in den großtechnischen Maßstab sowie die Weiterverarbeitung der Endprodukte erheblich erschweren.

Es besteht daher weiterhin ein Bedarf an verbesserten, großtechnisch durchführbaren Herstellungsverfahren für Chinolon-3-carbonsäuren der Formel I.

Der Erfindung lag somit die Aufgabe zu Grunde ein verbessertes Herstellungsverfahren für Chinolon-3-carbonsäuren der Formel I zur Verfügung zu stellen, welches die hohe Flexibilität hinsichtlich der Produktumstellung besitzt und die Produkte in hoher Ausbeute, Reinheit und Raum-Zeit-Ausbeute zur Verfügung stellt.

Die gestellte Aufgabe wird aufgrund der Merkmale des Anspruchs 1 gelöst und durch die weiteren Merkmale der Unteransprüche ausgestaltet und weiterentwickelt.

Es wurde überraschenderweise gefunden, dass die Verbindungen der Formel I, 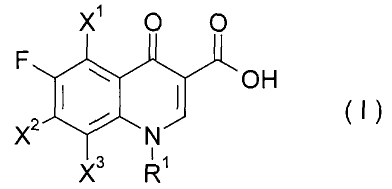 worin X¹, X², X³ und R¹ die angegebene Bedeutung besitzen, sowohl in hoher Ausbeute als auch in hoher Raum-Zeit-Ausbeute erhalten werden, wenn mindestens eine der Umsetzungen kontinuierlich geführt wird und die jeweiligen Edukte gegebenenfalls in Gegenwart eines inerten Verdünnungsmittels in einer Mischzone miteinander gemischt werden und in einer Reaktionszone gegebenenfalls unter erhöhtem Druck bei im wesentlichen konstanter Temperatur gehalten werden.

Ein weiterer Gegenstand der Erfindung ist ein Mikroreaktorsystem zur Durchführung eines mehrstufigen Verfahrens gemäß eines der vorangehenden Ansprüche bestehend aus zwei oder mehreren miteinander verbundenen Mikroreaktoren (13, 13'), welche jeweils mindestens zwei Eduktzuführungen (12) und eine Produktabführung (15) aufweisen, dadurch gekennzeichnet, dass mindestens ein Verweilzeitmodul (14) und/oder ein Reinigungsmodul zwischen einem ersten Mikroreaktor (13) und einem zweiten Mikroreaktor (13') geschaltet ist.

Vor- und nachstehend bedeutet der Begriff _{"}kontinuierlich" eine Verfahrensweise, bei der sich Reaktionsmedien in einem Durchfluss-System befinden, welches durch bestimmte Funktionszonen, insbesondere Mischzonen, Reaktionszonen und Verweilzonen geleitet wird. Dabei werden im Gegensatz zum diskontinuierlichen _{"}Batch"-Verfahren die Edukte in definierten Zeiteinheiten zugeführt und die Produkte in definierten Zeiteinheiten abgeführt.
Vor- und nachstehend bedeutet der Begriff _{"}Edukt" eine chemische Verbindung, die in einem nachfolgenden Reaktionsschritt weiterverarbeitet wird. Dieser Begriff umfasst sowohl chemische Verbindungen, die neu in den Gesamtprozess eingeführt werden als auch solche, die in einem vorgeschalteten Prozess erzeugt wurden und weiterverarbeitet werden.

Vor- und nachstehend bedeutet der Begriff _{"}Halogen" einen Substituenten ausgewählt aus der Gruppe, Fluor, Chlor, Brom und Jod, vorzugsweise Fluor oder Chlor, insbesondere Fluor.

Vor- und nachstehend bedeuten die Begriffe _{"}Alkyl", _{"}Alkoxy" und _{"}Haloalkyl" geradkettige oder verzweigte aliphatische Reste mit bis zu 6 Kohlenstoffatomen, vorzugsweise mit bis zu 3 Kohlenstoffatomen, insbesondere Methyl, Ethyl, Propyl, Isopropyl, Methoxy, Ethoxy, Propoxy, Trifluormethyl, 2,2,2-Trifluorethyl, Pentafluorethyl oder 2-Fluorethyl.

Vor- und nachstehend bedeutet der Begriff _{"}Cycloalkyl", einen cycloaliphatischen Rest mit 3 bis 8 Kohlenstoffatomen, vorzugsweise mit 3 bis 6 Kohlenstoffatomen, insbesondere Cyclopropyl, Cyclobutyl, Cyclopentyl oder Cyclohexyl, speziell Cyclopropyl.

Vor- und nachstehend bedeutet der Begriff _{"}Aryl" einen aromatischen Rest mit 6 bis 10 Kohlenstoffatomen, vorzugsweise Phenyl oder Naphthyl, insbesondere Phenyl.

Vor- und nachstehend bedeutet der Begriff _{"}Heteroaryl" einen heteroaromatischen Rest mit 5 oder 6 Ringglieder, der mindestens ein Heteroatom ausgewählt aus der Gruppe Stickstoff, Sauerstoff und Schwefel aufweist. Bevorzugt sind Thienyl-, Furanyl- und Azolylreste sowie Azine, insbesondere Thien-2-yl, Furan-2-yl, Pyrrolyl, Imidazolyl, Pyridazolyl, Oxazolyl, Thiazolyl, Thiadiazolyl, Pyridyl, Pyrimidyl, Pyrazinyl oder Triazinyl.

Vor- und nachstehend bedeutet der Begriff _{"}Heterocyclischer Rest" wie er für die Gruppe NR²R³ verwendet wird einen nichtaromatischen heterocyclischen Rest mit 3 bis 8 Ringgliedern, vorzugsweise 5 oder 6 Ringglieder, welcher gegebenenfalls ein oder zwei weitere Heteroatome aus der Gruppe O, S und N aufweist. Besonders bevorzugt sind Pyrrolidin-1-yl, Piperidin-1-yl, Piperazin-1-yl und Morpholin-1-yl.

Falls einer der genannten Reste X¹, X², X³, R¹, R² und R³ als eine gegebenenfalls substituierte Gruppe bezeichnet ist, kann diese Gruppe unsubstituiert oder mit einem oder der maximal möglichen Anzahl, vorzugsweise 1 bis 5, insbesondere 1 oder 2 Substituenten substituiert sein.

Diese Substituenten sind solche, die gegenüber den Reaktionsbedingungen des erfindungsgemäßen Verfahrens inert sind, vorzugsweise Fluor oder Chlor, Hydroxy, C₁-C₆-Alkyl, C₁-C₆-Alkoxy, C₁-C₆-Halolkyl, C₁-C₆-Halolkoxy, Amino, C₁-C₆-Alkylamino, Di-(C₁-C₆-Alkyl)-amino, C₁-C₆-Alkoxycarbonyl oder Phenyl.

Besonders bevorzugt ist ein Verfahren zur Herstellung der Verbindungen der Formel IA 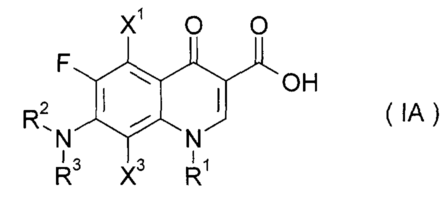 worin X¹, X³ und R¹ die angegebene Bedeutung besitzen, und
R² und R³ zusammen mit dem eingeschlossenen Stickstoffatom einen gegebenenfalls substituierten heterocyclischen Rest mit 5 oder 6 Ringgliedern und gegebenenfalls einem weiteren Heteroatom ausgewählt aus der Gruppe O, S und N bilden; insbesondere solche Verbindungen der Formel IA, wobei X¹ und X³ Wasserstoff bedeuten, und R¹ für eine C₃-C6-Cycloalkylgruppe steht.

Ganz besonders bevorzugt sind 1-Cyclopropyl-6-fluor-7-piperazin-1-yl-1,4-dihydro-4-oxo-chinoloncarbonsäure sowie 1-Cyclopropyl-6-fluor-7-(3-acetylamino-pyrrolid-1-yl)-1,4-dihydro-4-oxo-chinoloncarbonsäure.

In einer weiteren bevorzugten Ausführungsform des erfindungsgemäßen Verfahrens zur Herstellung der Verbindungen der Formel I werden Verbindungen der Formel II, 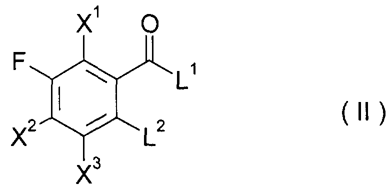 mit einer Verbindung der Formel III, 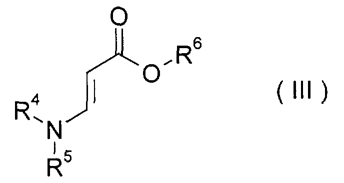 einer Verbindung der Formel IV,

H₂NR¹ (IV)

und gegebenenfalls einer Verbindung der Formel V,

HNR²R³ (V)

worin jeweils X¹, X², X³, R¹, R² und R³ die in Anspruch 1 angegebene Bedeutung besitzen, und
L¹ und L² jeweils unabhängig voneinander für eine geeignete Abgangsgruppen stehen, und R⁴, R⁵ und R⁶ jeweils unabhängig voneinander für eine C₁-C₆-Alkylgruppe oder Benzylgruppe stehen,
in geeigneter Reihenfolge in einem oder mehreren kontinuierlichen Verfahren miteinander zur Reaktion gebracht.

Die Erfindung wird im folgenden anhand der Zeichnungen und Ausführungsbeispielen näher beschrieben. Dabei zeigen:
Fig. 1a ein schematisches Flussbild zur Durchführung einer Synthesesequenz des erfindungsgemäßen Verfahrens
Fig. 1b ein schematisches Flussbild zur Durchführung von zwei kombinierten Synthesesequenzen des erfindungsgemäßen Verfahrens
Fig. 2a veranschaulicht den Aufbau eines Mikroreaktors zur Durchführung des erfindungsgemäßen Verfahrens
Fig. 2b veranschaulicht den Aufbau eines Mikroreaktors zur Durchführung des erfindungsgemäßen Verfahrens
Fig. 3a und 3b jeweils eine perspektivische Darstellung des inneren Formkörpers (1) des erfindungsgemäßen Verweilzeitmoduls in schematischer Darstellung.

Bei dem erfindungsgemäßen Verfahren werden die Edukte, insbesondere die Verbindungen der Formeln II, III, IV und gegebenenfalls V, jeweils in der Regel in einer für die Zuführung in den jeweiligen Mischer und/oder Reaktor geeigneten Form vorgelegt und über Dosierpumpen in die Mischer gefördert.

Geeignete Formen können Lösungen, Suspensionen oder Dispersionen der Edukte sein. Das reine Edukt kann auch direkt zudosiert werden, sofern es in flüssiger Form vorliegt. Die Ausgangsverbindungen können, je nach Anforderung gekühlt, erwärmt, gerührt, mit Ultraschall beschallt oder unter erhöhten oder verminderten Druck gesetzt werden. Zur Aufbereitung von Zwischenprodukten für weitere Reaktionsschritte können spezielle Module in der Anlage vorgesehen werden, z.B. Filtriervorrichtungen, Ultrafiltrationseinheiten Ionenaustauscher, Adsorber, Homogenisatoren, Verdampfer und/oder Extraktoren, welche vorzugsweise kontinuierlich betrieben werden.

Die Vorlagen, Pumpen, Reaktoren und Verweilzeitmodule können temperiert werden. Die Zuführung der Stoffe bzw. deren Zubereitungen in die Reaktoren erfolgt vorzugsweise über Pumpen. Alle gängigen Pumpentypen können Verwendung finden, insbesondere Kolbenpumpen. Membranpumpen, Zahnradpumpen oder Schlauchpumpen. Zur Dämpfung von Pulsationen können Pulsationsdämpfer eingesetzt werden.

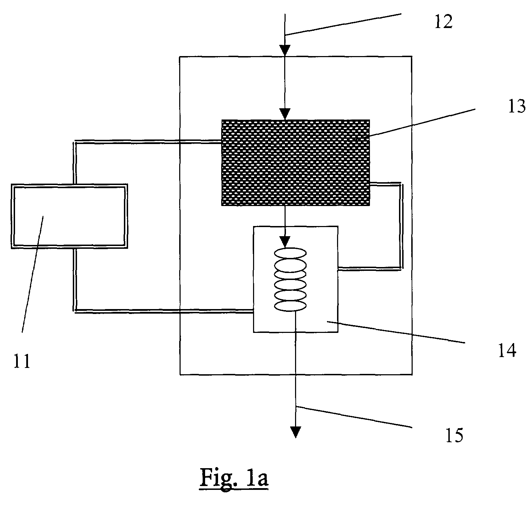

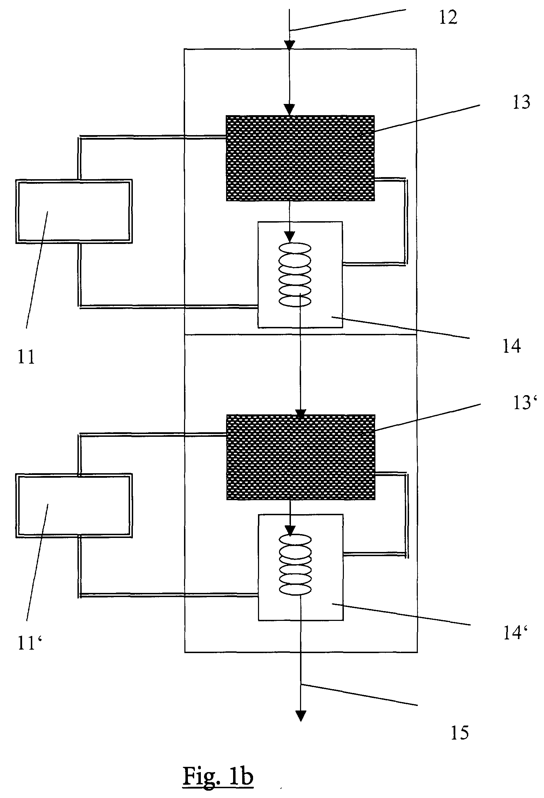

Die Funktionsweise der Vorrichtung zur Durchführung des erfindungsgemäßen Verfahrens wird anhand der Zeichnungen beschrieben. Dabei zeigt: Fig. 1a das Flussbild einer einzelnen Reaktionssequenz und Fig. 1b das Flussbild zweier miteinander verknüpfter Reaktionssequenzen. Der Eduktstrom (12) wird in die Mischer- und Reaktoreinheit (13) eingeleitet und nach Passieren eines optionelles Verweilzeitmoduls (14) zur Weiterverarbeitung (15) geleitet. Mischer- und Reaktoreinheit und das Verweilzeitmodul werden durch eine Temperatursteuerung (11) bei konstanter Temperatur gehalten. Der aus einem ersten Verweilzeitmodul (14) kommende Produktstrom wird zusammen mit einem weiteren Reagenz im Mischer- und Reaktoreinheit (13') zur Reaktion gebracht. Durch ein zweites Verweilzeitmodul (14') kann auch der zweiten Reaktionssequenz genügend Zeit zur thermischen Nachbehandlung (11') zur Verfügung gestellt werden.

Nach Ablauf jedes Reaktionsschrittes im Reaktorsystem tritt das Reaktionsprodukt kontinuierlich aus dem Reaktorausgang (15) aus und kann dann in eine für die Weiterverwendung geeignete Form gebracht werden, z.B. durch kontinuierliches Ausfällen und Abfiltrieren aus der Lösung, Destillation, Sprühtrocknung, Umkristallisation, usw..

Als kontinuierlicher Prozess wird die Synthese weitgehend automatisch gesteuert. Durch Prozessrechner werden sämtliche Abläufe überwacht und gesteuert. Geeignete Sensoren und Prozessrechner sind bekannt und kommerziell erhältlich.

Durch Variation der Edukte in einer oder mehrerer Synthesestufen können unterschiedliche Produkte erhalten werden. Bei dieser Vorgehensweise entfallen die sonst bei Produktumstellung erforderlichen Rüstzeiten oder werden zumindest erheblich verkürzt.

In einer besonders bevorzugten Ausführungsform des erfindungsgemäßen Verfahrens werden nacheinander folgende Schritte durchgeführt:
(a) Umsetzung der Verbindung der Formel II mit einer Verbindung der Formel III unter Ausbildung eines 2-Benzoyl-3-aminoacrylats der Formel VI 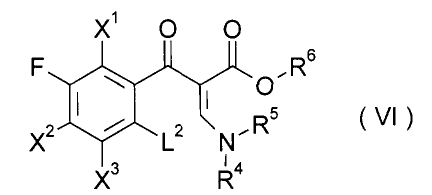
(b) Umsetzung der Verbindung der Formel VI mit einem primären Amin der Formel IV unter Ausbildung eines 2-Benzoyl-3-aminoacrylats der Formel VII 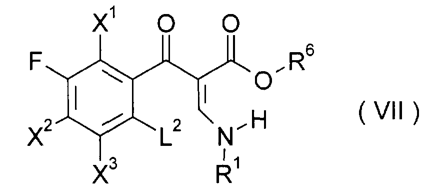
(c) Cyclisierung der Verbindung der Formel VII in Gegenwart einer Base unter Bildung eines Chinolon Derivats der Formel VIII
(d) 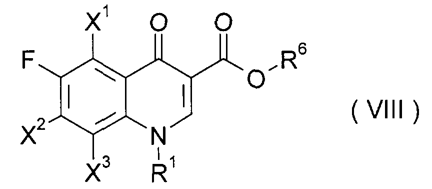
(e) gegebenenfalls Umsetzung der Verbindung der Formel VIII mit einer Verbindung der Formel V unter Bildung eines 7-substituierten Chinolon Derivats der Formel IX
(f) 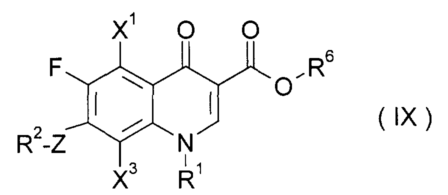
(g) Verseifung der Verbindung der Formel IX, und
(h) anschließendem Ansäuern, wobei das innere Salz der Verbindung der Formel I isoliert wird.

Die Umsetzung der Reaktionsschrittes (a) erfolgt vorzugsweise in Gegenwart eines inerten Verdünnungsmittels und bei erhöhter Temperatur, vorzugsweise bei Temperaturen von 30 bis 100 °C, insbesondere von 50 bis 70 °C, ganz besonders bevorzugt bei etwa 60 °C. Geeignete Verdünnungsmittel sind apolare Lösungsmittel, vorzugsweise gegebenenfalls halogenierte, aromatische oder aliphatische Kohlenwasserstoffe wie zum Beispiel Benzol, Toluol, Xylol, Dichlormethan oder Chloroform oder polar aprotische Lösungsmittel, vorzugsweise Amide wie zum Beispiel Dimethylformamid, Dimethylacetamid, N-Methylpyrrolidinon oder Harnstoffderivate wie zum Beispiel Tetramethylharnstoff oder Dimethylpropylenharnstoff oder Mischungen der genannten Verdünnungsmittel. Besonders bevorzugt ist Chloroform.

Die Umsetzung der Reaktionsschrittes (b) erfolgt vorzugsweise in Gegenwart eines inerten Verdünnungsmittels und bei Temperaturen von 0 bis 60 °C, insbesondere von 20 bis 60 °C, ganz besonders bevorzugt bei etwa 30 bis 40 °C. Bei diesen Temperaturen ist die Reaktion innerhalb von 1 bis 60 Minuten, vorzugsweise 3 bis 15 Minuten, insbesondere in etwa 5 Minuten in einem Mikroreaktor beendet. In einem herkömmlichen Rohrreaktor muss aufgrund der geringeren Durchmischung mit längeren Reaktionszeiten gerechnet werden. Geeignete Verdünnungsmittel sind apolare Lösungsmittel, vorzugsweise gegebenenfalls halogenierte, aromatische oder aliphatische Kohlenwasserstoffe wie zum Beispiel Benzol, Toluol, Xylol, Dichlormethan oder Chloroform oder protische Lösungsmittel, vorzugsweise C₁-C₄ Alkohole wie zum Beispiel Methanol, Ethanol oder Isopropanol, polar aprotische Lösungsmittel, vorzugsweise Amide wie zum Beispiel Dimethylformamid, Dimethylacetamid, N-Methylpyrrolidinon oder Harnstoffderivate wie zum Beispiel Tetramethylharnstoff oder Dimethylpropylenharnstoff oder Gemische der genannten Verdünnungsmittel. Besonders bevorzugt ist Chloroform.

Die Umsetzung der Reaktionsschrittes (c) erfolgt vorzugsweise in Gegenwart eines inerten, hochsiedenden Verdünnungsmittels und bei erhöhter Temperatur, vorzugsweise bei Temperaturen von 30 bis 160 °C, insbesondere von 50 bis 150 °C, ganz besonders bevorzugt in einem Temperaturbereich zwischen etwa 70 und 140 °C. Geeignete Verdünnungsmittel sind hochsiedende Lösungsmittel, vorzugsweise gegebenenfalls halogenierte, aromatische oder aliphatische Kohlenwasserstoffe wie zum Beispiel Xylol oder Tetrachlormethan oder polar aprotische Lösungsmittel, vorzugsweise Amide wie zum Beispiel N-Methylpyrrolidinon oder Harnstoffderivate wie zum Beispiel Tetramethylharnstoff oder Dimethylpropylenharnstoff, Ether wie zum Beispiel Dioxan, Alkohole wie zum Beispiel *tert*-Butanol oder *tert*-Amylalkohol oder Mischungen der genannten Verdünnungsmittel. Die Reaktion wird vorzugsweise in Gegenwart nichtnucleophiler Basen, vorzugsweise von Metallalkoxiden wie zum Beispiel Natrium oder Kalium *tert*-Butoxid, Metalldialkylamiden, wie zum Beispiel Lithiumdiisopropylamid oder Kaliumhexamethyldisilazan, tertiären Aminen wie zum Beispiel 4-Dimethylamino-2,6-dimethylpyridin oder 1,8-Diazobicyclo[5.4.0]undec-7-en (DBU) durchgeführt. Im Anschluss an Reaktionsschritt (c) werden vorzugsweise die gebildeten Ammoniumsalze abgetrennt. Die Abtrennung wird in der Regel durch Extraktion mit Wasser, durch Ausfällen in der Kälte und Filtration oder bevorzugt durch Verdampfen der flüchtigen Stoffe und Extraktion des Produktes aus dem Rückstand mit Hilfe eines selektiv lösenden Lösungsmittels, vorzugsweise N-Methylpyrrolidon durchgeführt.

Die Umsetzung der Reaktionsschrittes (d) erfolgt vorzugsweise in Gegenwart eines inerten, hochsiedenden Verdünnungsmittels und bei erhöhter Temperatur, vorzugsweise bei Temperaturen von 30 bis 160 °C, insbesondere von 50 bis 150 °C, ganz besonders bevorzugt in einem Temperaturbereich zwischen etwa 70 und 140 °C. Geeignete Verdünnungsmittel sind hochsiedende Lösungsmittel, vorzugsweise gegebenenfalls halogenierte, aromatische oder aliphatische Kohlenwasserstoffe wie zum Beispiel Xylol oder Tetrachlormethan oder polar aprotische Lösungsmittel, vorzugsweise Amide wie zum Beispiel N-Methylpyrrolidinon oder Harnstoffderivate wie zum Beispiel Tetramethylharnstoff oder Dimethylpropylenharnstoff, Ether wie zum Beispiel Dioxan, Alkohole wie zum Beispiel *tert*-Butanol oder *tert*-Amylalkohol oder Mischungen der genannten Verdünnungsmittel. Die Reaktion wird vorzugsweise in Gegenwart eines Überschusses der Verbindung der Formel V und einer Hilfs-Base, vorzugsweise von Metallalkoxiden wie zum Beispiel Natrium oder Kalium tert-Butoxid, Metalldialkylamiden, wie zum Beispiel Lithiumdiisopropylamid oder Kaliumhexamethyldisilazan, tertiären Aminen wie zum Beispiel 4-Dimethylamino-2,6-dimethylpyridin oder 1,8-Diazobicyclo[5.4.0]undec-7-en (DBU) durchgeführt.
Der intramolekulare Ringschluss des Reaktionsschrittes (c) verläuft wesentlich schneller als die Einführung des Restes R2-X- in der 7-Position des Reaktionsschrittes (d). Durch geeignete Temperaturwahl für Reaktor- und Verweilzonen können die Schritte (c) und (d) in einem stufenweise in einem Reaktor durchgeführt werden.

Die Verseifung in Schritt (e) erfolgt in der Regel in an sich bekannter Weise mit einer wässrigen oder wässrig-alkoholischen Base bei einer Temperatur von 50 bis 120 °C, vorzugsweise 60 bis 110 °C, insbesondere bei etwa 70 bis 90 °C.

Nach Abfiltration fester Nebenprodukte erhält man durch Ansäuern der Reaktionslösung vorzugsweise mit einer Mineralsäure wie zum Beispiel Salzsäure, Salpetersäure oder Schwefelsäure das innere Salz der Verbindung der Formel I.
Als Reaktoren werden vorzugsweise Mikroreaktoren (13, 13') eingesetzt.

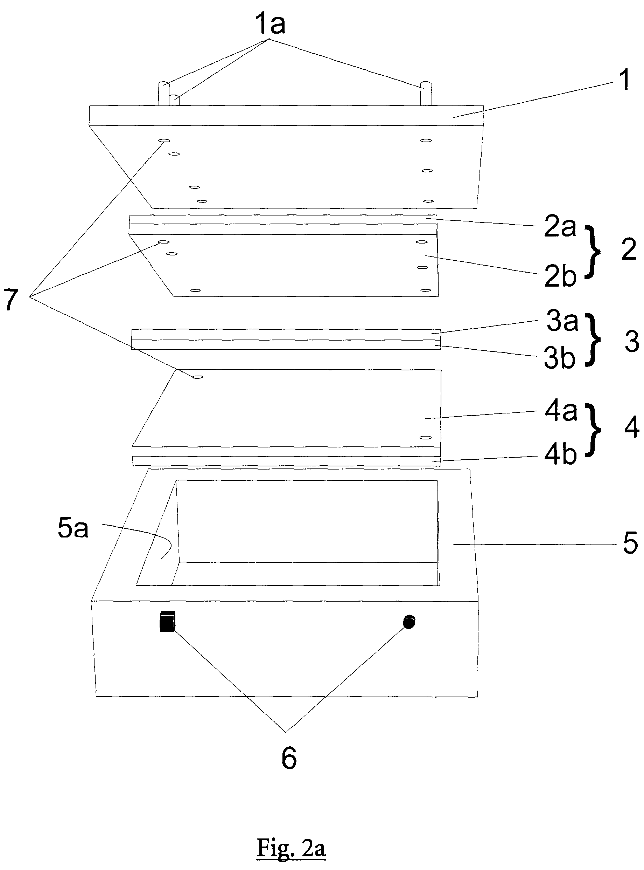

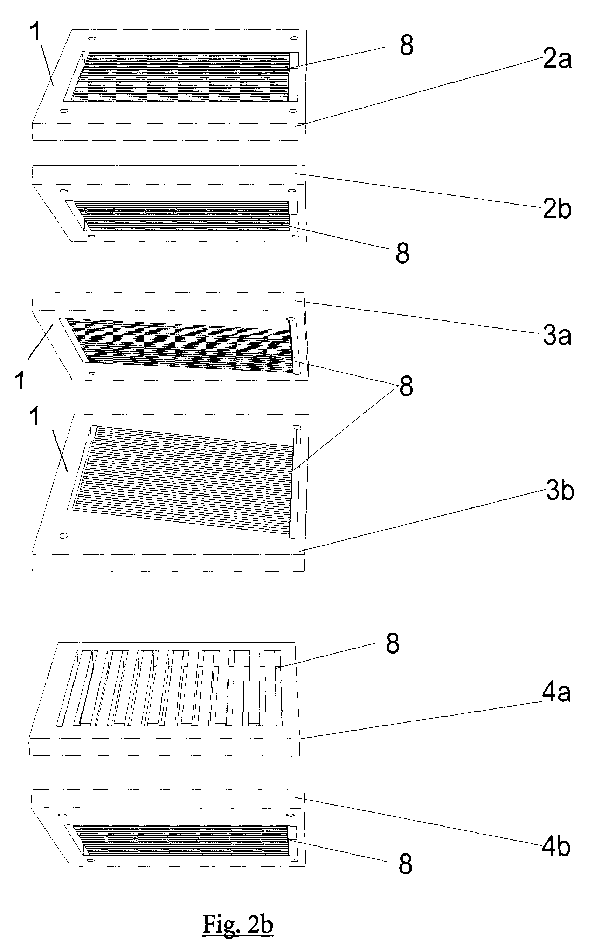

Solche Mikroreaktoren werden zum Beispiel in der EP 0 688 242-B, der US 5,811,062 oder der europäischen Patentanmeldung Nr. EP 00103012.1 beschrieben. Die Funktionsweise eines Mikroreaktors gemäß der EP 00103012.1 wird beispielhaft in den Fig. 2a und 2b dargestellt. Dieser Mikroreaktor wird anhand der Zeichnungen beschrieben. Dabei zeigt: Fig. 2a einen Mikroreaktor in schematischer, auseinander gezogener Darstellung und Fig. 2b vergrößerte Einzelheiten des Mikroreaktors.

Zwischen einem Gehäusedeckel 1 und einem Gehäuse 5 sind eine Anzahl von Funktionsmodulen 2, 3 und 4 angeordnet, die durch den Zusammenbau unter Druck gehalten werden, um Abdichtflächen zwischen den Modulen zusammenzupressen. Jedes Funktionsmodul 2, 3 und 4 enthält eine Modulhälfte 2a, 2b bzw. 3a, 3b bzw. 4a, 4b, die jeweils rahmenartig gestaltet sind, um Abdichtflächen 10 darzubieten, die beim Aufeinanderpressen der Hälften abdichten. Im Deckel sind Fluidanschlüsse 1a angebracht, die sich in Fluidkanälen 7 durch die Randbereiche der Funktionsmodule fortsetzen. Von dort gibt es horizontale Kanäle zu Funktionsräumen 8, die in der Regel ein Kanalsystem oder Labyrinthsystem beinhalten. Die Kanäle der Funktionsräume 8 laufen im allgemeinen schräg oder quer zueinander.

Die aus zwei Hälften zusammengebauten Funktionsmodule zeigen an ihren Oberflächen jeweils Öffnungen der Kanäle 7, die in standardisierten Abständen angeordnet sind, um beim Stapelaufbau von Funktionsmodulen genau zueinander zu fluchten. Diese Öffnungen sind mit Dichtstrukturen versehen, um fortlaufende Kanäle 7 abdichtend zu kuppeln. Diese Dichtstrukturen können als zylindrische Stutzen mit kegelförmigen oder sphärischen Abdichtflächen ausgebildet und genügend nachgiebig sein, damit die plattenförmigen Funktionsmodule mit ihrem gesamten Randbereich aufeinander liegen, um den Pressdruck auf die innenliegenden Abdichtflächen 10 zu übertragen.

Im dargestellten Ausführungsbeispiel sei angenommen, dass das Funktionsmodul 2 einen Wärmetauscher darstellt, der aus einer Wärmetauscherhälfte 2a für Kühl- und/oder Heizmedium und einer Wärmetauscherhälfte 2b für Reaktantenführung besteht. Das Funktionsmodul 3 sei ein Mischer für einen Reaktionspartner A und einen Reaktionspartner B. Das Funktionsmodul 4 stellt eine Verweilstrecke dar, die aus einer Verweilstreckenhälfte 4a für Reaktionsprodukt und einer Verweilstreckenhälfte 4b für Kühl- und/oder Heizmedium besteht.

Vorzugsweise weisen die Mikrostrukturen im Bereich der Reaktanten- und der Reaktionsgemisch-Führung Kanalbreiten von 400-1000 µm, insbesondere 500-700 µm und Kanalhöhen von 200-800 µm, insbesondere 400-600 µm auf, und die Mikrostrukturen weisen im Bereich der Wärmeträger-Führung Kanalbreiten von 100-800 µm, insbesondere 300-600 µm und Kanalhöhen von 100-400 µm, insbesondere 200-300 µm auf, wobei nur Geometrien verwendet werden, die keine Totwasserzonen ausbilden.

Da solche Mikroreaktoren gewöhnlich kontinuierlich betrieben werden, ist es nicht empfehlenswert, das aus dem Mikroreaktor kommende Reaktionsgemisch in einen herkömmlichen Reaktor zu füllen und dort verweilen zu lassen.

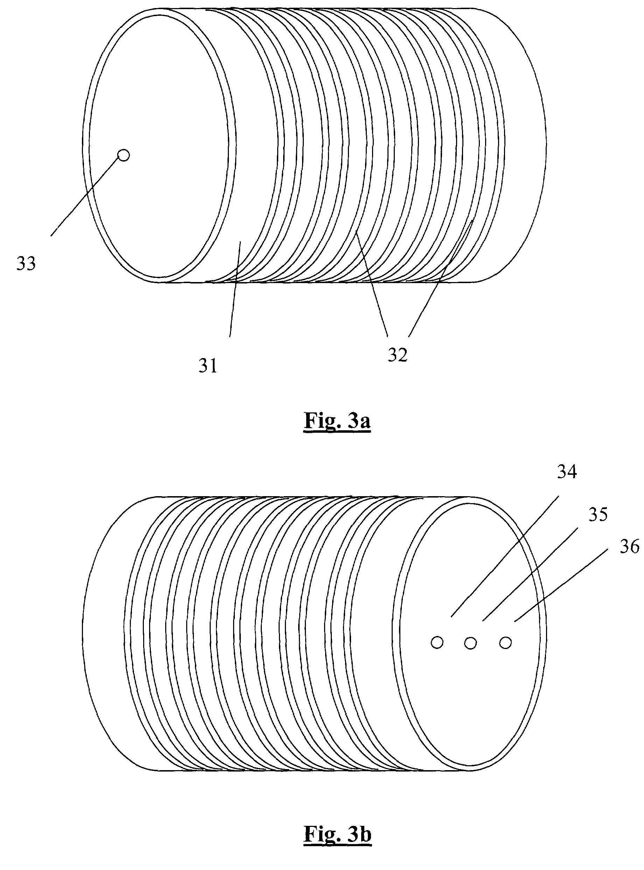

Ein optimales Verweilzeitmodul ist schematisch in Fig. 3a und 3b dargestellt.

Dieses Verweilzeitmodul zur thermischen Nachbehandlung chemischer Reaktionsmedien aus Mikroreaktoren weist folgende Bestandteile auf:
(a) einen Formkörper (31) mit mindestens einer zwei- oder dreidimensional gewundenen Vertiefung (32), die sich auf mindestens einer Oberfläche des Formkörpers befindet, eine durch den Formkörper führende Öffnung (33), durch welche die mindestens eine Vertiefung mit einem Reaktionsmedium beschickt werden kann, und eine oder mehrere Öffnungen (34, 35, 36) zur wahlweisen Entnahme des Reaktionsmediums;
(b) eine über die strukturierte Oberfläche bzw. die strukturierten Oberflächen des Formkörpers gelegte Abdeckung, welche die mindestens eine Vertiefung dichtend abschliesst und so mindestens einen kanalförmigen Hohlraum ausbildet;
(c) gegebenenfalls Vorrichtungen zur Temperaturregulierung und/oder Sensoren zur Kontrolle des Reaktionsverlaufs.

Der Begriff Verweilzeit bedeutet vorstehend und nachfolgend die Zeit, die dem Reaktionsmedium zur Nachreaktion nach der Durchmischung in dem erfindungsgemäßen Modul zur Verfügung gestellt wird. In der Regel ist sie abhängig von der Durchflussgeschwindigkeit und dem Volumen der zwei- oder dreidimensional gewundenen, bevorzugt spiral- oder schraubenförmigen Vertiefung auf mindestens einer Seite des Formkörpers. Das Volumen der Vertiefung lässt sich durch die Länge und den Durchmesser des Moduls, die Ganghöhe der schraubenförmigen Windung sowie die geometrische Lage der Produktentnahmeöffnung vorbestimmen.

Das erfindungsgemäße Verfahren erlaubt es die Verbindungen der Formel 1, insbesondere 1-Cyclopropyl-6-fluor-7-piperazin-1-yl-1,4-dihydro-4-oxo-chinoloncarbonsäure in guter Ausbeute und Reinheit herzustellen. Durch Parallelisierung, größere Reaktionsvolumina, Arbeiten bei höheren Temperaturen oder unter erhöhtem Druck können die Durchsatzraten noch weiter gesteigert werden. Der Prozess lässt sich weitgehend automatisieren und ist kontinuierlich durchführbar. Daher ist der erfindungsgemäße Prozess wesentlich wirtschaftlicher durchzuführen als die arbeitsintensiven herkömmlichen Batch-Verfahren.

Um das Verständnis der vorliegenden Erfindung zu erleichtern werden die nachfolgenden illustrativen Beispiele für mögliche Reaktionstypen dargelegt. Die vorliegende Erfindung ist nicht beschränkt auf diese spezifischen Ausführungsformen, sondern umfasst den vollen Umfang der Patentansprüche.

### Anwendungsbeispiele

### Beispiel 1

Herstellung von 1-Cyclopropyl-6-fluor-7-piperazin-1-yl-1,4-dihydro-4-oxo-chinoloncarbonsäure
1A 2-(2,4,5-Trifluorbenzoyl)-3-dimethylaminoacrylsäureethylester
   Ein Gemisch aus Dimethalaminoacrylsäureethylester (1,25 mol/l), Triethylamin (1,4 mol/l) und Chloroform wird mit 0,12 ml/min in einen Mikroreaktor der mit einer 50 ml fassenden Verweilzeitkapillare versehen ist, gepumpt. Über eine zweite Pumpe wird mit 0,12 ml/min ein Gemisch von 2,4,5-Trifluorbenzoylchlorid (1,3 mol/l) und Chloroform in den Reaktor gepumpt. Die Temperatur in Reaktor und Verweilzeitkapillare wird bei 60 °C gehalten.
1B 2-(2,4,5-Trifluorbenzoyl)-3-cyclopropylaminoacrylsäureethylester
   Nach Passieren der Verweilzone wird die 1A-haltige Reaktionsmischung bei einer Temperatur von 35 °C in einem weiteren Reaktor mit einem Gemisch aus Eisessig (1,5 mol/l), Cyclopropylamin (1,43 mol/l) in einem Verhältnis von 2:1 vemischt. Die Produktlösung wird aufgefangen, Ammoniumsalze und Lösungsmittel werden abgetrennt und das Zwischenprodukt 1B in N-Methylpyrrolidinon verdünnt (200 g/l).
1C 1-Cyclopropyl-6,7-difluor-1,4-dihydro-4-oxo-chinolon-3-carbonsäureethylester
   Das Gemisch von 1B (200 g/l) und N-Methylpyrrolidinon wird in einem weiteren Mikroreaktor bei einer Temperatur von 120 °C mit einem Gemisch von DBU (230 g/l) und N-Methylpyrrolidinon in einem Verhältnis von 2:1 über Dosierpumpen (0,16 ml/min bzw. 0,08 ml/min) gemischt. Nach Durchgang über eine 15 ml Verweilzeitkapillare wird das erhaltene Produkt in einen weiteren Mikroreaktor mit einer 50 ml Verweilzeitkapillare gepumpt.
1D 1-Cyclopropyl-6-fluor-7-piperazin-1-yl-1,4-dihydro-4-oxo-chinolon-3-carbonsäureethylester
   Das in 1C erhaltene Gemisch wird bei 85 °C mit einem Gemisch aus Piperazin (250 g/l), Triethylamin (100 g/l), tert-Butanol und N-Methylpyrrolidinon mit 0,16 ml/min vermischt.
1E 1-Cyclopropyl-6-fluor-7-piperazin-1-yl-1,4-dihydro-4-oxo-chinolon-3-carbonsäure
   Das in 1D erhaltene Gemisch wird bei 85 °C in einen weiteren Mikroreaktor mit einer Verweilzeitkapillare von 53 ml mit 0,40 ml/min gepumpt und mit einem Gemisch von 24 g/l Natriumhydroxid und Wasser gemischt. Die erhaltene Reaktionsmischung wird mit Weser verdünnt, filtriert und mit wässrigen Natriumhydrogencarbonat und Salzsäure auf einen pH Wert zwischen 7 und 8 eingestellt. Dabei fällt spontan das innere Salz von 1E als gelbes Pulver aus. Dieses wird isoliert, mit Wasser gewaschen und getrocknet.
   Die Verbindung 1E (3,38 g = 68 % d. Th.) wird nach 100 Minuten Betriebszeit als reines Produkt erhalten.

### Beispiel 2

Herstellung von 1-Cyclopropyl-6-fluor-7-piperazin-1-yl-1,4-dihydro-4-oxochinoloncarbonsäure
2A 1-Cyclopropyl-6,7-difluor-1,4-dihydro-4-oxo-chinolon-3-carbonsäureethylester
   Das Gemisch von 1B (Beispiel 1, 200 g/l) und N-Methylpyrrolidinon wird in einem weiteren Mikroreaktor bei einer Temperatur von 120 °C mit einem Gemisch von Kalium *tert*-Butoxid (230 g/l) und *tert*-Butanol in einem Verhältnis von 2:1 über Dosierpumpen (0,16 ml/min bzw. 0,08 ml/min) gemischt. Nach Durchgang über eine 5 ml
   Verweilzeitkapillare wird das erhaltene Produkt in einen weiteren Mikroreaktor mit einer 50 ml Verweilzeitkapillare gepumpt. Die Weiterverarbeitung erfolgt analog Beispiel 1. Man erhält das reine Produkt 1E in hoher Ausbeute (65 % d. Th.) in einer Betriebszeit von 90 Minuten.

### Beispiel 3

Herstellung von 1-Cyclopropyl-6-fluor-7-piperazin-1-yl-1,4-dihydro-4-oxochinoloncarbonsäure
3A 1-Cyclopropyl-6-fluor-7-piperazin-1-yl-1,4-dihydro-4-oxo-chinolon-3-carbonsäureethylester
   Der zweistufige Prozess aus baseninduzierter Cyclisierung (1C) und nucleophiler Substitution mit Piperazin (1D) wird durch geeignete Temperaturführung in verschiedenen Reaktionszonen eines Mikroreaktors gesteuert. Die Cyclisierung wir bei einer Temperatur zwischen 60 und 80 °C in einer ersten Reaktionszone durchgeführt und die Substitution bei einer Temperatur zwischen 80 und 90 °C in einer nachgeschalteten Zone.

### Beispiele 4 bis 9

Die Verbindungen der Formeln 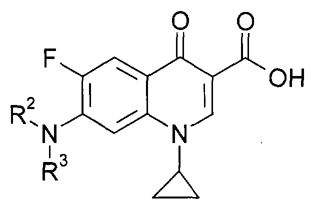 werden in Analogie zu den in den Beispielen 1 bis 3 beschriebenen Verfahren hergestellt:

| Beispiel | R² | R³ |
|---|---|---|
| 4 | C₄H₉ | C₄H₉ |
| 5 | -(CH2)5- | |
| 6 | -(CH₂)₂-O-(CH₂)₂- | |
| 7 | -(CH₂)₂-CH(CH₃)-(CH₂)₂- | |
| 8 | -(CH2)4- | |
| 9 | -(CH₂)-CH(NH-CO-CH₃)-(CH₂)₂- | |

## Patentansprüche

1. Verfahren zur Herstellung der Verbindungen der Formel I, 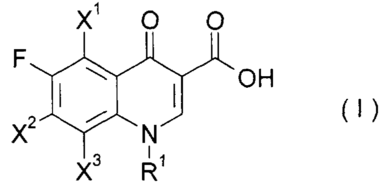 worin
X¹ für Wasserstoff, Halogen, C₁-C₆-Alkyl oder Amino steht,
X² für Halogen, C₁-C₆-Alkyl, gegebenenfalls substituiertes C₆-C₁₀-Aryl, gegebenenfalls substituiertes 5- oder 6-gliedriges Stickstoffhaltiges Heteroaryl steht, oder eine Gruppe der Formel ZR² bedeutet, wobei Z für O, S oder NR3 steht, und R² und R³ jeweils unabhängig voneinander C₁-C₆-Alkyl, gegebenenfalls substituiertes C₆-C₁₀-Aryl, gegebenenfalls substituiertes 5- oder 6-gliedriges Stickstoffhaltiges Heteroaryl bedeuten, oder R² und R³ zusammen mit dem eingeschlossenen Stickstoffatom einen gegebenenfalls substituierten heterocyclischen Rest mit 3 bis 8 Ringgliedern und gegebenenfalls einem oder zwei weiteren Heteroatomen ausgewählt aus der Gruppe O, S und N bilden,
X³ für Wasserstoff, Halogen, C₁-C₆-Alkyl, C₁-C₆-Alkoxy oder Amino steht, und
R¹ für C₁-C₆-Alkyl, C₁-C₆-Haloalkyl, C₃-C₈-Cycloalkyl, gegebenenfalls substituiertes C₆-C₁₀-Aryl, oder gegebenenfalls substituiertes 5- oder 6-gliedriges Stickstoffhaltiges Heteroaryl steht,
**dadurch gekennzeichnet, dass** mindestens eine der Umsetzungen kontinuierlich geführt wird und die jeweiligen Edukte gegebenenfalls in Gegenwart eines inerten Verdünnungsmittels in einer Mischzone miteinander gemischt werden und in einer Reaktionszone gegebenenfalls unter erhöhtem Druck und bei im wesentlichen konstanter Temperatur gehalten werden.

2. Verfahren nach Anspruch 1, **dadurch gekennzeichnet, dass** die Verbindung der Formel II, 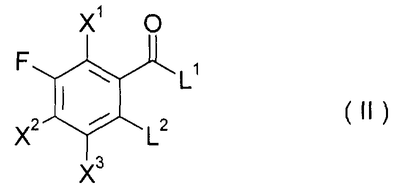 mit einer Verbindung der Formel III, 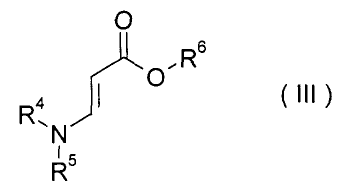 einer Verbindung der Formel IV,
H₂NR¹ (IV)
und gegebenenfalls einer Verbindung der Formel V,
HZR² (V)
worin jeweils X¹, X², X³, R¹, R² und Z die in Anspruch 1 angegebene Bedeutung besitzen, und
L¹ und L² jeweils unabhängig voneinander für eine geeignete Abgangsgruppen stehen, und R⁴, R⁵ und R⁶ jeweils unabhängig voneinander für eine C₁-C₆-Alkylgruppe oder Benzylgruppe stehen,
in geeigneter Reihenfolge in einem oder mehreren kontinuierlichen Verfahren miteinander zur Reaktion bringt.

3. Verfahren nach Anspruch 1 oder 2, **dadurch gekennzeichnet, dass** das Reaktionsgemisch durch mehrere hintereinandergeschaltene Reaktoren geschickt wird, denen an einer oder mehreren Stellen weitere Reagenzien hinzugeführt werden.

4. Verfahren nach einem der vorangehenden Ansprüche, **dadurch gekennzeichnet, dass** das jeweils in einer Reaktionssequenz erhaltene Reaktionsgemisch vor seiner Weiterverarbeitung einen Reinigungsschritt durchläuft.

5. Verfahren nach Anspruch 4, **dadurch gekennzeichnet, dass** der Reinigungschritt die Abtrennung von Nebenprodukten, die Aufkonzentrierung, die erneute Verdünnung und/oder Filtration der Reaktionslösung umfasst.

6. Verfahren nach einem der vorangegangen Ansprüche, **dadurch gekennzeichnet, dass** nacheinander folgende Schritte durchgeführt werden:
(a) Umsetzung der Verbindung der Formel II mit einer Verbindung der Formel III unter Ausbildung eines 2-Benzoyl-3-aminoacrylats der Formel VI 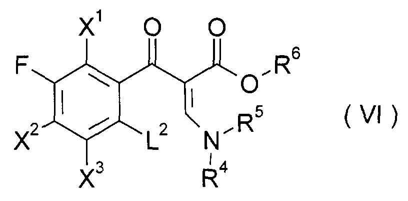
(b) Umsetzung der Verbindung der Formel VI mit einem primären Amin der Formel IV unter Ausbildung eines 2-Benzoyl-3-aminoacrylats der Formel VII 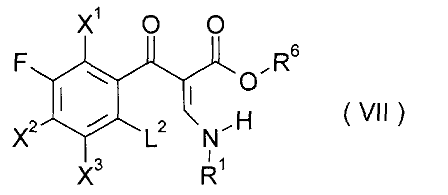
(c) Cyclisierung der Verbindung der Formel VII in Gegenwart einer Base unter Bildung eines Chinolon Derivats der Formel VIII 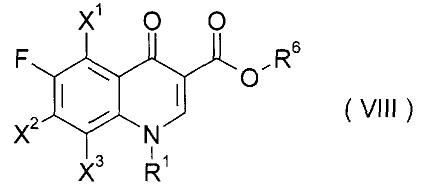
(d) gegebenenfalls Umsetzung der Verbindung der Formel VIII mit einer Verbindung der Formel V unter Bildung eines 7-substituierten Chinolon Derivats der Formel IX 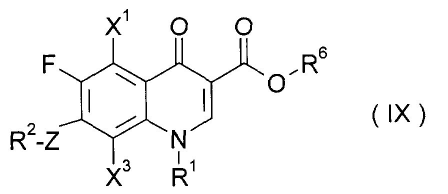
(e) Verseifung der Verbindung der Formel IX, und
(f) anschließendem Ansäuern, wobei das innere Salz der Verbindung der Formel I isoliert wird.

7. Verfahren nach Anspruch 6, **dadurch gekennzeichnet, dass** die in Reaktionschritt (b) anfallenden Salze abgetrennt werden und das Reaktionsgemisch nach Reaktionsschritt (e) filtriert wird.

8. Verfahren nach einem der vorangehenden Ansprüche, **dadurch gekennzeichnet, dass** in einem oder mehreren Reaktionsschritten ein Mikroreaktor eingesetzt wird.

9. Verfahren nach einem der vorangehenden Ansprüche **dadurch gekennzeichnet, dass** die Reaktion in einer oder mehreren Vorrichtungen durchgeführt wird, die Pumpen, Reaktoren (8), Verweilzeitmodule (9), temperierbare Zu- und Ableitungen umfassen.

10. Verfahren nach einem der vorangehenden Ansprüche **dadurch gekennzeichnet, dass** die Reaktion in einer oder mehreren Mikroreaktoren durchgeführt wird, wobei Platten oder Schichten (2a, 2b, 3a, 3b, 4a, 4b) so übereinander gestapelt sind, dass sich Kanäle und horizontal erstreckende Räume (6) bilden, in denen die physikalischen und chemischen Funktionen durchgeführt werden.

11. Verfahren nach Anspruch 7, **dadurch gekennzeichnet, dass** die Mikrostrukturen im Bereich der Reaktanten- und der Reaktionsgemisch-Führung Kanalbreiten von 400-1000 µm und Kanalhöhen von 200-800 µm aufweisen, und die Mikrostrukturen im Bereich der Wärmeträger-Führung Kanalbreiten von 100-800 um und Kanalhöhen von 100-400 µm aufweisen und nur Geometrien verwendet werden, die keine Totwasserzonen ausbilden.

12. Verfahren nach einem der vorangehenden Ansprüche, **dadurch gekennzeichnet, dass** die Reaktion in einem oder mehreren Mikroreaktoren durchgeführt wird, die als integrierte Funktionsmodule Mischer, Wärmetauscher, Verweilstrecken, Filter, Verdampfer, Destillationskolonnen, Ionenaustauscher, Ultrafiltrationszellen und/oder Extraktionskolonnen aufweisen.

13. Verfahren nach einem der vorangehenden Ansprüche, **dadurch gekennzeichnet, dass** die Reaktion in einer oder mehreren Vorrichtungen durchgeführt wird, die Sensoren zur Erfassung der Prozessparameter wie Temperatur, Druck, Strömungsgeschindigkeit, Volumen, Massestrom und/oder pH-Wert aufweisen.

14. Verfahren nach Anspruch 13, **dadurch gekennzeichnet, dass** die Reaktion in einer oder mehreren Vorrichtungen durchgeführt wird, in der aufgrund der gemessenen Prozessparameter der Materialfluss in den fluidischen Anschlüssen und die Energiezufuhr und -abfuhr geregelt wird.

15. Ein Mikroreaktorsystem zur Durchführung eines mehrstufigen Verfahrens gemäß eines der vorangehenden Ansprüche bestehend aus zwei oder mehreren miteinander verbundenen Mikroreaktoren (13, 13'), welche jeweils mindestens zwei Eduktzuführungen (12) und eine Produktabführung (15) aufweisen, **dadurch gekennzeichnet, dass** mindestens ein Verweilzeitmodul (14) und/oder ein Reinigungsmodul zwischen einem ersten Mikroreaktor (13) und einem zweiten Mikroreaktor (13') geschaltet ist.

16. Mikroreaktorsystem nach Anspruch 15, **dadurch gekennzeichnet, dass** die Mikroreaktoren Platten oder Schichten (2a, 2b, 3a, 3b, 4a, 4b) aufweisen, die so übereinander gestapelt sind, dass sich Kanäle und horizontal erstreckende Räume (6) bilden, in denen die physikalischen und chemischen Funktionen durchgeführt werden können.
